# EUROPEAN PATENT APPLICATION

(11) **EP 3 406 275 A1**
(43) Date of publication of application: **28.11.2018**
(21) Application number: 17461536.9
(22) Date of filing: 22.05.2017
(51) Int. Cl.: A61M 1/00, A61F 13/00, A61F 13/02

(54) **WOUND TREATMENT APPARATUS**

(71) Applicant: Unipad Sp. z o.o., 60-476 Poznan (PL)
(72) Inventor: Banasiewicz, Tomasz, 62-005 Czerwonak (PL)
(74) Representative: Kancelaria Eupatent.pl Sp. z.o.o

(57) **Abstract**

A wound treatment apparatus comprising: a connection hub (11, 21) comprising a first side and a second side; an adhesion pad (16, 26) for attaching the connection hub to the vicinity of the wound; and a plurality of tubes (12-15; 22-24) having internal ends (12B-15B; 22B-24B) for inserting into the wound and external ends (12A-15A; 22A-24A) for attaching the wound treatment apparatus to external entities.

## Description

### TECHNICAL FIELD

The present invention relates to a wound treatment apparatus, in particular to a wound treatment apparatus adapted to work with negative pressure.

### BACKGROUND

Vacuum Assisted Closure (VAC) is a closed system utilizing a negative pressure in the wound bed. It has many advantages, widely recognized in the art. In particular, the vacuum assisted closure: promotes cleaning wounds from secretions and toxic products; prevents accumulation of fluids in deeper layers of wounds, thereby accelerating the process of wound healing; increases vascularity, thereby improving microcirculation; limits bacterial growth in the wound; stimulates wounds to granulate; decreases swelling around the wound; enhances perfusion of the wound bed; stimulates proliferation of fibroplasts and endothelial cells; removes the excess of effusion from the wound surface; provides moisture to the wound; reduces amount of bacteria on the wound surface.

Negative pressure therapy systems known in the art are usually electrically powered, costly and require a lot of space. They have to be operated by a qualified personnel. These systems are usually dependent on a single model of a product and can be used only in form of complete, closed systems. Liquids can be supplied only in the immediate vicinity of foams, and the space which can be rinsed is relatively limited. In most cases only one channel is provided for supplying the liquid. There is no possibility for simultaneously providing oxygen, monitoring the process of healing, obtaining samples for tests.

There is a need to provide an improved and cost effective apparatus for implementation with vacuum dressings, which is simple in construction and versatile in application.

The system proposed herein connects vacuum therapy with the possibility of instillation (fluid or liquids with healing potential "injection"), oxygen supplementation (direct oxygen supplementation to improve the healing conditions, as well as eliminate the anaerobic pathogenic bacterial flora) and testing of biochemical condition of the wound (fluid aspiration to further analysis).

### SUMMARY

There is disclosed a wound treatment apparatus comprising: a connection hub comprising a first side and a second side; an adhesion pad for attaching the connection hub to the vicinity of the wound; a plurality of tubes having internal ends for inserting into the wound and external ends for attaching the wound treatment apparatus to external entities.

The tubes can be channeled through the connection hub.

The adhesion pad can be attached at the internal side to the connection hub.

The internal end of at least one of the tubes can be perforated.

The apparatus may comprise a fluid supply tube for supplying fluid to the volume of the wound.

The apparatus may comprise a fluid offtake tube for drawing off the wound content.

The apparatus may comprise an analysis tube for analyzing selected parameters of the wound.

The apparatus may comprise a receiving tube for receiving wound excretion.

At least one tube can be channeled through the walls of the receiving tube which forms the connection hub.

The internal ends of the tubes can be surrounded by a sponge.

The external ends of the tubes may have a conical shape.

There is also disclosed a method for preparing for use a wound treatment apparatus as described above, the method comprising: determining dimensions of a wound; cutting the internal ends of the tubes to a length depending on the determined dimensions of the wound.

### BRIEF DESCRIPTION OF FIGURES

These and other objects of the invention presented herein, are accomplished by providing wound treatment apparatus. Further details and features of the present invention, its nature and various advantages will become more apparent from the following detailed description of the preferred embodiments shown in a drawing, in which:
Fig. 1 presents a VAC system known in the art;
Fig. 2 presents a module according to a first embodiment;
Fig. 3 presents a module according to a variant of the first embodiment;
Fig. 4 presents a module according to a second embodiment;
Fig. 5 presents a module according to a variant of the second embodiment;

### DETAILED DESCRIPTION

The invention provides a universal module compatible with known and commonly used vacuum dressings, as well as other system or direct use of the hospital's vacuum system (called a "poor man vacuum"). All advantages of negative-pressure wound therapy are utilized, while introducing new elements enhancing wound healing. The invention can allow to influence wound healing process to a greater extent and can provide a wider spectrum of possibilities for healing depending on individual prescriptions. An important feature is introduction to a standard system (a single drain to evacuate the exudation form the wound) further catheters/drains that give the possibility to increase the effectiveness of the therapy by simple instillation (wound washing), oxygen supply, performing biochemical and microbiological tests.

The module can be adapted according to medical requirements (clinical situation), which is demonstrated by showing two embodiments.

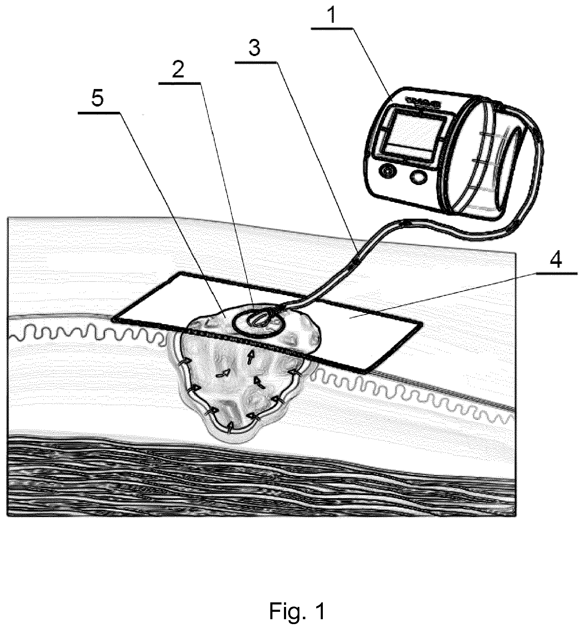

Fig. 1 shows a known negative vacuum wound treatment system known in the art. It comprises a vacuum dressing 4 (drape), which is applied onto a wound in which a foam 5 has been placed. The vacuum dressing 4 comprises a connector 2, which is connected with a negative pressure source 1 by means of a tube 3. Upon activating the source 1, a negative pressure is produced under the drape 4, which aids wound healing.

The apparatus presented herein has a "low-profile" system, which makes its use, more comfortable for patient and avoids system blockage.

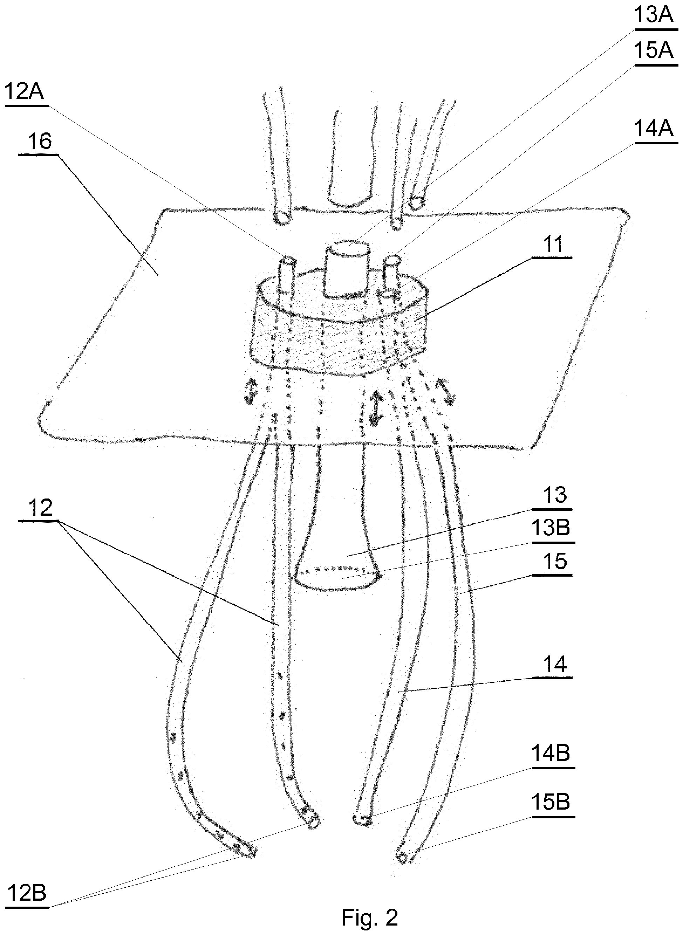

Fig. 2 shows the first embodiment of the module. The module comprises a universal connection hub 11 (made according to methods known in the art for hubs), which serves as a base for a plurality of drains. The number of drains protruding from the port depends on the medical needs. The module comprises an adhesion film 16 around the connection hub 11, which facilitates its attaching to its destination point, for example to vacuum dressing, and producing negative pressure.

There are fluid supply tubes 12 for supplying fluid from an external end 12A to the internal end 12B positioned within the wound. The internal end 12B may be split to a plurality of tubes and/or may have perforated walls, to aid supplying fluid to the whole volume of the wound.

The module further comprises a receiving tube 13 for receiving the wound excretion from an internal end 13B forming a receiving drain to be placed within the wound to an external end 13A. Its internal end 13B diameter can be selected depending on the wound dimensions. The receiving tube 13 can be used to protect fistulas or complicated ostomy. The internal end 13B of the receiving tube 13 may have a conical shape (expanding in a direction away from the hub), so that it can be cut at a desired position to adapt its diameter to the wound dimensions.

The module also comprises a fluid offtake tube 14, for drawing off the wound fluid or content from an internal end 14B forming an offtake drain to be placed within the wound to an external end 14B.

Further, there is provided a small diameter analysis tube 15, for analyzing selected parameters of the wound (e.g. the pH) via an internal drain 15B to be placed within the wound to an external end 15B.

To the bottom side (i.e. the side from which the drains protrude into the wound) there is also attached an adhesion plate 16 for attaching to a vacuum dressing.

The module can be provided in a variety of sizes and/or different lengths of drains. Alternatively, a module with long drains can be provided and the drains can be cut to desired length depending on the wound dimensions. The depth of insertion of the drains into the wound can be regulated by cutting them to a desired length.

The external ends 12A-15A of the tubes 12-15, located at the top side of the connection hub 11 are to be connected with external devices. The external ends may have a conical shape, so that their end diameter can be regulated by cutting them at a selected place, so as to comply with the connecting tubes of external devices.

The first embodiment is particularly useful for treating "shallow" wounds (wherein the surface dimensions of the wound are much bigger than its depth). The pad has a relatively large diameter, which increases the effectiveness of the therapy of superficial wounds. Examples of such wounds include venous ulcerations, diabetic foot, bedsores.

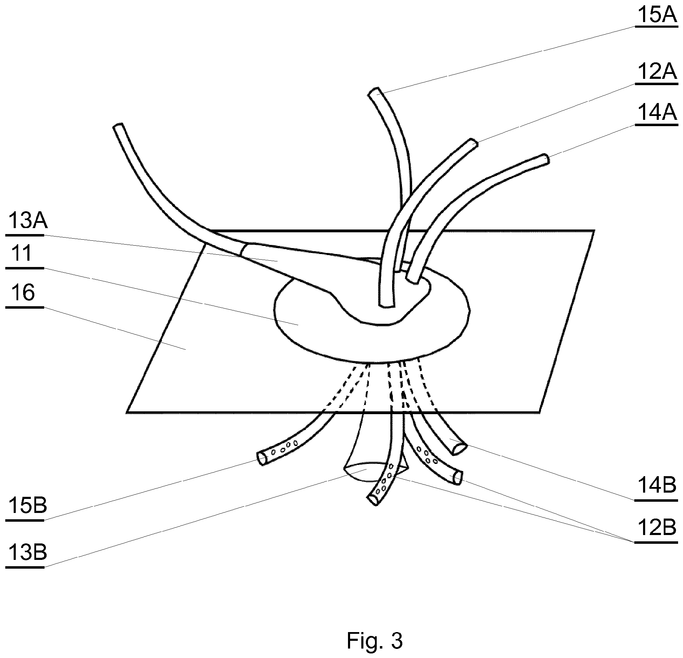

Fig. 3 shows a variant of the first embodiment, wherein the body 11 of the connection hub is shaped such that the external end 13A of the receiving tube 13 is positioned such that its axis is inclined at an acute angle to the main surface, in particular substantially horizontal to the main surface (i.e. the surface of the body within which the would is present). This may facilitate the connection of the receiving tube 13 to external collection devices.

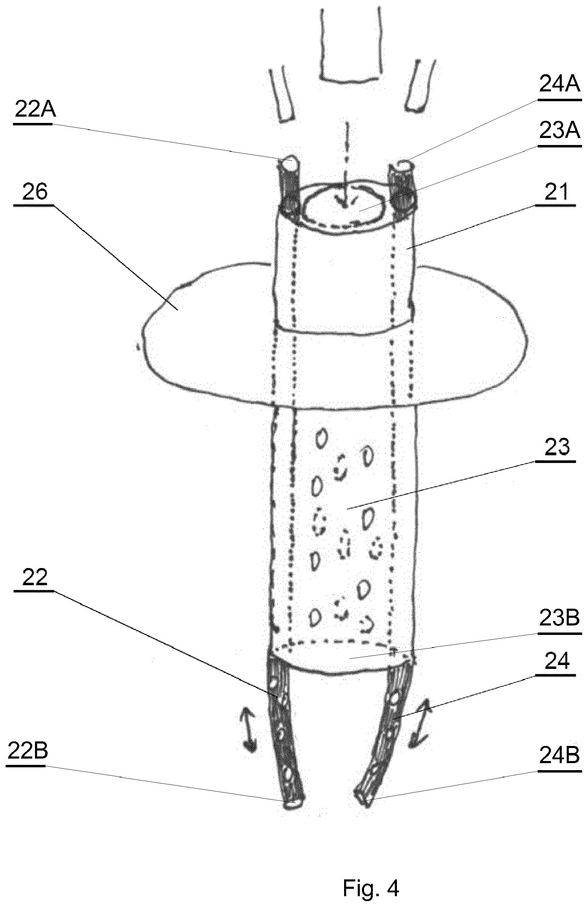

Fig. 4 shows a second embodiment of the module. It comprises a universal connection hub 21.

There are fluid supply tubes 22 protruding from the connection hub 21. They can be perforated, which aids supplying fluid to the whole volume of the wound from an external end 22A to an internal end 22B.

The module further comprises a receiving tube 23, having an external end 23A and an internal end 23B which can be perforated. The receiving drain 23 can be used to protect fistulas or complicated ostomy.

The module further comprises a fluid offtake tube 24, for drawing off the wound fluid or content from an internal end 24B serving as a drain to an external end 24A.

The depth of insertion of the tubes into the wound can be regulated, for example by cutting them to a desired length.

To the bottom side of the connection hub 21 (i.e. the side from which the drains protrude) there is also attached an adhesion plate 26 for attaching to a vacuum dressing. The top side of the connection hub 21 comprises external ends of the tubes to be connected with external devices, as described with respect to the first embodiment.

The body of the receiving tube 23 can constitute the connection hub 21 and have a cylindrical shape, wherein the remaining tubes are channeled through and run along the walls of the receiving tube 23. The receiving tube 23 can be perforated, wherein multiple perforations increase the effectiveness of exudation evacuation. This allows more precise control of the module when it is inserted into a deep wound, which reduces a risk of further damage. This system also connects vacuum with possibility of instillation, and improves the effectiveness of suction by multiple perforations in the drain.

The second embodiment can further comprise a sponge surrounding the internal ends 22B-24B of the tubes 22-24 (not shown for clarity). The sponge can be made of PVA (polyvinyl alcohol). This material assures lack of adhesion to tissues, which leads to elimination of the risk of interaction. Such effect is especially advantageous when the wound comprises blood vessels, nerves, bowel walls etc.

The second embodiment is particularly useful for treating "deep" wounds (wherein the depth of the wound is much bigger than its surface dimensions). This type of wound, as a fistula, is difficult to treat with standard negative pressure methods, wherein a sponge should be inserted deep into the wound, but using only a sponge (without a drain) can be ineffective. The module of the second embodiment gives the possibility to insert the deep drain into different types of sponges. Connection of the sponge with the proposed drain can be done by simple surgical sutures, easily and commonly done by surgeons. This embodiment can operate with any negative-pressure system or can work as a stand-alone device, connected directly to any vacuum pump used in negative-pressure therapy.

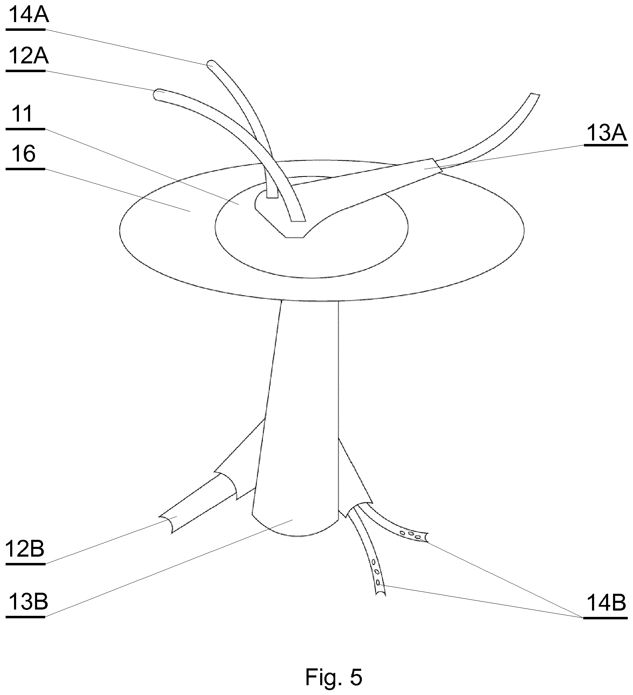

Fig. 5 shows a variant of the second embodiment, wherein the body 21 of the connection hub is shaped such that the external end 23A of the receiving tube 23 is positioned such that its axis is inclined at an acute angle to the main surface, in particular substantially horizontal to the main surface (i.e. the surface of the body within which the would is present). This may facilitate the connection of the receiving tube 23 to external collection devices.

The tubes can serve to provide agents that stimulate healing, eliminate biofilm (unfavorable spatial structure caused by bacteria, which increases their resistance to antibiotics) and of antiseptic functions. By using the tubes, both fluids and gases can be supplied to the wound. The fluids can be commercially available preparations or mixtures prepared specifically for the treatment. An important example of gases to be provided is oxygen, which allows for implementing elements of hyperbaric therapy, which is very efficient in treatment of difficult wounds. The tubes can also allow to retrieve material from the wound in order to determine wound healing progress. The tubes can further enable pH testing, which is a sensitive parameter enabling wound healing progress, which also can be used to determine a necessity of replacing the dressing. The modules presented herein are also useful for wound instillation, by the tubes inserted to the volume of the wound. The modules presented herein are compatible with typically used wound dressings and typical vacuum therapy pumps.

## Claims

1. A wound treatment apparatus comprising:
- a connection hub (11, 21) comprising a first side and a second side;
- an adhesion pad (16, 26) for attaching the connection hub to the vicinity of the wound; and
- a plurality of tubes (12-15; 22-24) having internal ends (12B-15B; 22B-24B) for inserting into the wound and external ends (12A-15A; 22A-24A) for attaching the wound treatment apparatus to external entities.

2. The apparatus according to any of previous claims, wherein the tubes (12-15; 22-24) are channeled through the connection hub (11, 21).

3. The apparatus according to any of previous claims, wherein the adhesion pad (16, 26) is attached at the internal side to the connection hub (11, 21).

4. The apparatus according to any of previous claims, wherein the internal end (12A-15A; 22B-24B) of at least one of the tubes (12-15; 22-24) is perforated.

5. The apparatus according to any of previous claims, comprising a fluid supply tube (12, 22) for supplying fluid to the volume of the wound.

6. The apparatus according to any of previous claims, comprising a fluid offtake tube (14, 24) for drawing off the wound content.

7. The apparatus according to any of previous claims, comprising an analysis tube (15) for analyzing selected parameters of the wound.

8. The apparatus according to any of previous claims, comprising a receiving tube (13, 23) for receiving wound excretion.

9. The apparatus according to claim 8, wherein at least one tube (22, 24) is channeled through the walls of the receiving tube (23) which forms the connection hub (21).

10. The apparatus according to any of previous claims, wherein the internal ends (12A-15A, 22B-24B) of the tubes (12-15; 22-24) are surrounded by a sponge.

11. The apparatus according to any of previous claims, wherein the external ends (12A-15A; 22A-24A) of the tubes (12-15; 22-24) have a conical shape.

12. A method for preparing for use a wound treatment apparatus according to any of claims 1-11, the method comprising:
- determining dimensions of a wound;
- cutting the internal ends (12B-15B; 22B-24B) of the tubes (12-15; 22-24) to a length depending on the determined dimensions of the wound.
